(19) Europäisches Patentamt
European Patent Office
Office européen des brevets

(11) **EP 2 518 078 B1**

(12) **EUROPEAN PATENT SPECIFICATION**

(45) Date of publication and mention of the grant of the patent:
**20.05.2020 Bulletin 2020/21**

(21) Application number: **09852326.9**

(22) Date of filing: **15.12.2009**

(51) Int Cl.:
*C07K 1/10* *(2006.01)*     *C07K 1/107* *(2006.01)*
*A61P 35/00* *(2006.01)*     *A61K 47/68* *(2017.01)*
*C07K 1/02* *(2006.01)*

(86) International application number:
**PCT/KR2009/007510**

(87) International publication number:
**WO 2011/074717 (23.06.2011 Gazette 2011/25)**

(54) **METHOD FOR MANUFACTURING DIMERS AND MULTIMERS BY INCREASING THE PRODUCTION OF BOND BRIDGES IN A COMPLEX OF MULTIPLE MONOMERS AND REPEATING CHAINS OF AN AFFINITY DOMAIN OF A TYPE SPECIFICALLY BINDING TO PROTEIN MONOMERS**

VERFAHREN ZUR HERSTELLUNG VON DIMEREN UND MULTIMEREN MITTELS STEIGERUNG DER BILDUNG VON BINDUNGSBRÜCKEN IN EINEM KOMPLEX AUS MEHREREN MONOMEREN UND SICH WIEDERHOLENDEN KETTEN EINES BINDUNGSPROTEINS MIT SPEZIFISCHER BINDUNG AN DIE MONOMERE

PROCÉDÉ DE FABRICATION DE DIMÈRES ET DE MULTIMÈRES PAR AUGMENTATION DE LA PRODUCTION DE PONTS DE LIAISON DANS UN COMPLEXE DE MONOMÈRES MULTIPLES ET DE CHAÎNES RÉCURRENTES D'UN ADHÉSIF D'UN TYPE ADHÉRANT SPÉCIFIQUEMENT À DES MONOMÈRES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**31.10.2012 Bulletin 2012/44**

(73) Proprietor: **Choe, Muhyeon**
**Seocho-gu, Seoul 137-775 (KR)**

(72) Inventors:
• **LEE, YongChan**
**Asan-si, Chungcheongnam-do 336-190 (KR)**
• **WON, JaeSeon**
**Seoul 120-865 (KR)**

(74) Representative: **Grünecker Patent- und Rechtsanwälte**
**PartG mbB**
**Leopoldstraße 4**
**80802 München (DE)**

(56) References cited:
**EP-A1- 1 246 925     WO-A1-2005/000902**

WO-A1-2005/000902     WO-A2-2008/035217
US-A1- 2005 048 512     US-A1- 2007 092 933
US-B1- 6 492 498

• **DEYEV S M ET AL: "Design of multivalent complexes using the barnase-barstar module", NATURE BIOTECHNOLOGY, NATURE PUBLISHING GROUP, NEW YORK, NY, US, vol. 21, no. 12, 1 December 2003 (2003-12-01), pages 1486-1492, XP002372739, ISSN: 1087-0156, DOI: 10.1038/NBT916**
• **WILLUDA J ET AL: "Tumor targeting of mono-, di-, and tetravalent anti-p185(HER-2) miniantibodies multimerized by self-associating peptides", JOURNAL OF BIOLOGICAL CHEMISTRY, AMERICAN SOCIETY FOR BIOCHEMISTRY AND MOLECULAR BIOLOGY, US, vol. 276, no. 17, 27 April 2001 (2001-04-27), pages 14385-14392, XP002315802, ISSN: 0021-9258**

- **PARK J H ET AL: "A divalent recombinant immunotoxin formed by a disulfide bond between the extension peptide chains", MOLECULES AND CELLS, SEOUL, KR, vol. 12, no. 3, 31 December 2001 (2001-12-31), pages 398-402, XP009115675, ISSN: 1016-8478**
- **PARK J H., ET AL.: 'A divalent recombinant immunotoxin formed by a disulfide bond between the extension peptide chains' MOLECULES AND CELLS vol. 12, no. 3, 2001, pages 398 - 402, XP009115675**
- **HO S N ET AL: "Site-directed mutagenesis by overlap extension using the polymerase chain reaction", GENE, ELSEVIER, AMSTERDAM, NL, vol. 77, no. 1, 15 April 1989 (1989-04-15), pages 51-59, XP025737079, ISSN: 0378-1119, DOI: 10.1016/0378-1119(89)90358-2 [retrieved on 1989-04-15]**

<110> CHOE, MuHyeon

<120> High yield production methods for dimer and multimers formed by cross-linking bond bridge by increasing the bond bridge formation utilizing the complex of monomers and tandem-repeat chain of molecule with binding specificity

<130> 9fpo-11-42

<160> 2

<170> KopatentIn 1.71

<210> 1
<211> 49
<212> DNA
<213> Artificial Sequence

<220>
<223> P1 primer

<400> 1
gggcatatgc atcaccatca ccatcacacc ggtacaccag ccgtgacaa          49

<210> 2
<211> 45
<212> DNA
<213> Artificial Sequence

<220>
<223> P2 primer

<400> 2
cccgaattct tatccggacc cgcctccacc ttcagttacc gtaaa          45

**Description**

BACKGROUND OF THE INVENTION

1. Field of the Invention

The present invention relates to a method for manufacturing dimers and multimers from protein monomers based on increased yield. To achieve this, repeat chain-multiple monomer complexes are produced by using repeat chains of affinity domains specifically binding to protein monomers, and multimers are produced by utilizing the fact that inter-monomeric bond bridges are easily formed between monomers within the complexes. That is, the present invention relates to a method for manufacturing large volume of dimers and multimers by maximizing the formation of repeat chain-multiple monomer complexes by using repeat chains as a scaffold, and by increasing the formation of inter-monomeric disulphide bond bridges among monomers within the formed repeat chain-multiple monomer complexes.

2. Description of the Related Art

Antibody-toxin is produced by adding toxin to the antibody specifically binding to cancer cells (Pastan I. et al., Annu Rev Med. 58(2007) 221-237). Deyev et al. (Nature Biotechnology (2003), 21(12), pages 1486-1492)describe the formation of multimers by preparing repeat chains of barnase and mixing with monomers of barstar fused to scFv fragments. However, no covalent bonds are formed between the barstar monomers.

Monoclonal antibody (MAb), B3 binds to carbohydrate antigen (LeY) over-expressed on the surface of colon, stomach, ovary, breast, or pulmonary cancer (L.H. Pai, et al., Proc Natl Acad Sci U S A 88(1991) 3358-3362; I. Pastan, et al., Cancer Res. 51(1991) 3781-3787). PE38 is one of the derivatives of Psudomonas exotoxin (PE) (J. Hwang, et al., Cell. 48(1987) 129-136; V.K. Chaudhary, et al., J Biol Chem 265(1990) 16306-16310).

Fab, a fragment having antigen binding part of antibody, includes Fd chain (VH and CH1) and light chain (VL and CL). Fab antibody-toxin includes Fd chain (VH and CH1) and light chain (VL and CL), too. Fab-toxin has two advantages compared to single chain Fv (scFv)-toxin. First, the yield of Fab-toxin is 10 times higher than that of scFv-toxin (J. Buchner, et al., Biotechnology (N Y). 9(1991) 157-162; J. Buchner, et al., Biotechnology 10(1992) 682-685). Second, stability of Fab-toxin is improved in plasma of mouse (M. Choe, et al., Cancer Res. 54(1994) 3460-3467).

It has been reported that divalent Fab-toxin dimers made by inter-monomeric disulphide bond bridge have higher cytotoxicity than monovalent scFv-toxin (S.H. Choi, et al., Bull. Kor. Chem. Soc. 22(2001) 1361-1365; J.H. Park, et al., Mol Cells 12(2001) 398-402; M.H. Yoo, et al., J. Microbiol. Biotechnol. 16(2006) 1097-1103). Disulphide dimers are manufactured by disulphide bond of intra-monomer counterpartless Cystein (Cys) residues from two Fab-toxin monomers. The intra-monomer counterpartless cystein residues are located between Fab domain and PE38 of Fab-toxin monomers.

Dimeric (divalent) antibody-toxin is expected to have more advantages than monomeric (monovalent) antibody-toxin. For example, it has higher avidity since two antigen binding domains of dimers increase the strength of binding. Also, since two of toxin domains are transferred at the same time to the target cells by antibody-toxin of divalent dimers, it is possible that antibody-toxin of divalent dimers is able to kill the target cells with higher cytotoxicity than antibody-toxin of monovalent monomers. Furthermore, Fab-toxin has higher stability than scFv-toxin which often used to produce recombinant antibody-toxin (D. Rothlisberger, et al., J Mol Biol 347(2005) 773-789).

[B3(Fab)-ext-PE38]$_2$, a disulphide bond of B3(Fab)-ext-PE38 monomer, can be manufactured by fusing Fab domains of monoclonal antibody B3 and PE38 which is a derivative of Psudomonas Exotoxin A (KR 10-0566091), and it is reported that [B3(Fab)-ext-PE38]$_2$ has 11 times higher cytotoxicity than B3(Fab)-ext-PE38 when tested on CRL-1739 stomach cancer culture cell line (J.H. Park, et al., Mol Cells 12(2001) 398-402) .

Conventionally, disulphide bonded dimers were purified after refolding (S.H. Choi, et al., Bull. Kor. Chem. Soc. 22(2001) 1361-1365; J.H. Park, et al., Mol Cells 12(2001) 398-402; M.H. Yoo, et al., J. Microbiol. Biotechnol. 16(2006) 1097-1103). In previous reports, the refolding process of disulphide bonded dimers gave the yield about 0.014% ~ 0.25%. During the refolding process, these disulphide dimers were formed by random accessing of intra-monomer counterpartless cystein residues located between Fab domains and PE38 of antibody-toxin of two monomers. Although the yields of disulphide dimers have been increased through the improvements of refolding processes, the yield of dimers were extremely low considering that the yield of Fab-toxin, the mainly produced molecule during the folding process, reached to almost 10%. Since antibody-toxin monomers does not have self-binding-affinity among themselves, bond bridged dimers are created by random collisions between intra-monomer counterpartless cystein residues of antibody-toxin monomers during the refolding process. That is, low collision frequency among the intra-monomer counterpartless cystein residues results the inefficient formation of inter-monomer disulphide bond bridges between the accessing cystein residues from monomers during the refolding process, and thus the yield of dimers, created during the refolding process, is extremely low.

As a result, inventors have tried various methods to produce disulphide bonded dimers including methods for producing disulphide dimers from antibody-toxin monomer by recycling of oxidation-oxidation, oxidation-reduction and chemical cross-linker, but with these methods, the yield of dimers could not be improved.

Therefore, in search for a method to produce more [B3(Fab)-ext-PE38]$_2$, a dimer in which monomer B3(Fab)-ext-PE38 was disulphide bond bridged, the inventors constructed repeat chains which are recombinant proteins wherein Fab binding domains of Streptococcal protein G were repeated more than two times, and with using this chain as scaffold, Fab-toxin multimer complexes, which had multiple monomer antibody-toxins are bounded simultaneously thereto, were produced. In previous studies, it has been reported that the third domain (domain III) of protein G is able to bind to Fab fragment of IgG, and CH1 domain of Fab fragment has high binding affinity toward the domains of protein G (J.P. et al., Nature 359(1992) 752-754). As comparing to the refolding process, the repeat chains of the present invention have the following advantages: High binding affinity for monomer antibody-toxin is shown to the domains of the repeat chains. In this regard, local concentration of monomer antibody-toxin is high within repeat chains-multiple monomers complexes created by repeat chains. Increased local concentration brings high collision frequency among intra-monomer counterpartless cystein residues of each monomer antibody-toxin and acceleration of disulphide bond between monomers. Therefore, it is possible to produce large quantity of inter-monomer disulphide bond bridged dimers. Typical example of similar effect of the repeat chains of this invention is increase of enzyme reaction rate by proximity effect in enzyme reaction kinetics (Nicholas C. Price and Lewis Stevens. Fundamentals of Enzymology, 3rd Ed. Oxford University Press). As a result, the inventors were able to easily manufacture the antibody-toxin multimer complexes by simply mixing the repeat chains with monomer antibody-toxins, and completed the present invention by confirming that large quantity of [B3(Fab)-ext-PE38]$_2$ are produced through oxidation and reduction shuffling reactions of intra-monomer counterpartless cystein residues of monomer antibody-toxin within the complexes to accelerate disulphide bond of cystein residues between Fab domains and PE38.

SUMMARY OF THE INVENTION

The invention is defined by the claims. Any subject-matter falling outside the scope of the claims is provided for information purposes only. The present invention aims to provide a method for mass-producing multimers from monomers with inter-monomer bond bridges between monomers, with using repeat chains of monomer-specific affinity domains as scaffold to attach the monomers and to mass-produce multimer complexes in form of the repeat chain-multiple monomer complexes and forming the inter-monomer bridge bond between monomers in the repeat chain-multiple monomer complexes, and this provides higher and maximized production methods of multimers by the increased collision frequency between the intra-monomer counterpartless cystein residues in each monomers and subsequently facilitated formation of the inter-monomer disulphide bond bridges between the monomers compared to the low yield conventional method for producing inter-monomer bridge bonded multimers.

In order to achieve the object explained above, the present invention provides a method for producing inter-monomer bond bridged multimers including steps of:

  1) preparing repeat chains of affinity domains binding specifically to monomers;
  2) preparing repeat chain/multiple-monomer complexes of the repeat chains and the monomers with mixing the repeat chains of step 1) and the monomers; and
  3) separating multimers in which inter-monomer bond bridges are formed between monomers within the repeat chain/multiple-monomer complexes of step 2).

In an embodiment of this method of dimer formation in which antibody-toxin monomers are linked by disulphide bond bridges, the yield of inter-monomer disulphide bond bridge dimers of the antibody-toxin monomers increased to about 208 fold of the yield of the conventionally-reported refolding method, and thus the dimer formation yield was increased very high. The dimers produced according to the present invention have higher antigen binding strength, higher cytotoxicity and higher stability than the previously-reported monomer antibody-toxin, and particularly show approximately 11 times higher cytotoxicity to stomach cancer cell line. Therefore, mass production of the dimers can be effectively used for the development of cancer treatment agents.

BREIF DESCRIPTION OF THE DRAWINGS

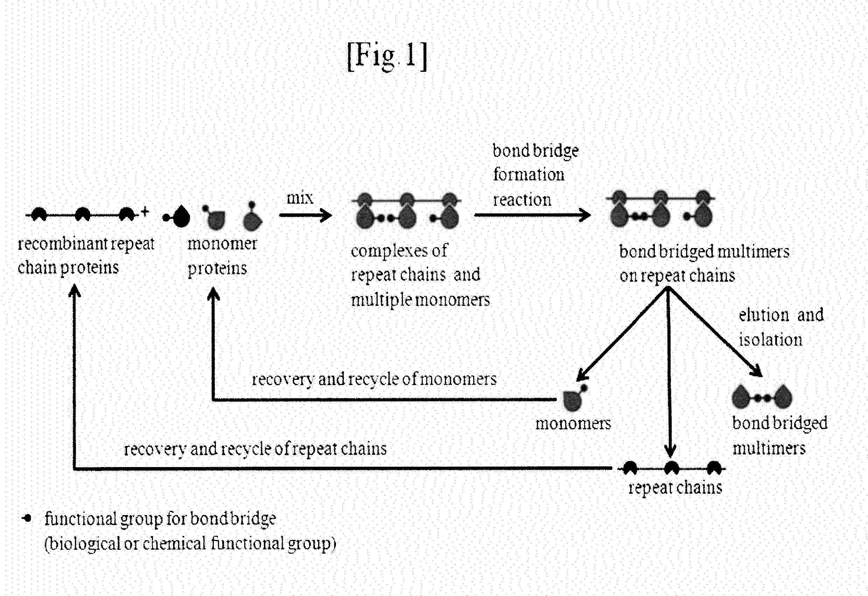

  FIG.1 presents outline of a method for producing multimers with bond bridges according to the present invention;
  FIG.2 presents the result of the experiments in which the purified proteins of the present invention are confirmed with SDS-polyacrylamide gel, in which:

FIG.2A presents the result of the experiments in which recombinant repeat chain proteins of protein G are mixed with SDS-sample buffer solution, and the mixture is analyzed in reducing 16% SDS-Polyacrylamide gel, wherein the lanes of No. 1 to 7 are TR1~TR7 respectively; and

FIG.2B presents the result of analyzing B3(Fab)-ext-PE38 (Monomer) and [B3(Fab)-ext-PE38]2(Dimer) antibody-toxin molecules, produced according to the present invention, in non-reducing 8% SDS-Polyacrylamide gel, in which each arrow indicates [B3(Fab)-ext-PE38]2, B3(Fab)-ext-PE38 and B3(Fd)-ext-PE38 from the top (Since (H6-B3(L) has very small molecular weight (i.e., about 25kDa), this cannot be seen in the drawings); and

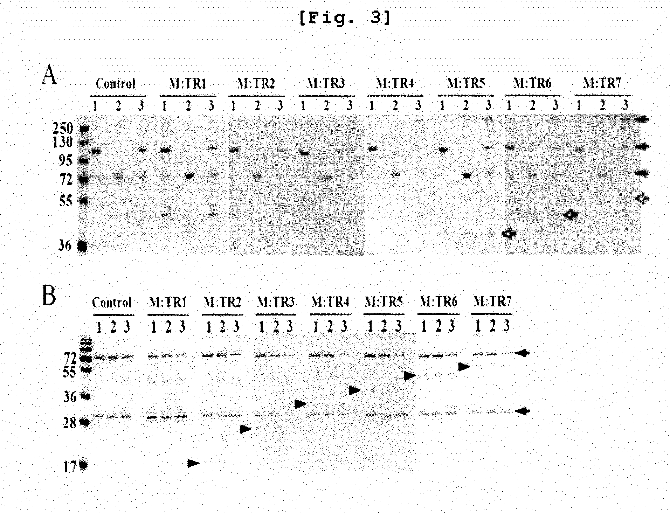

FIG.3 presents the result of analyzing the [B3(Fab)-ext-PE38]2 that were formed in the complexes of B3(Fab)-ext-PE38 and TR1 ~ TR7 proteins, by SDS-Polyacrylamide gel, in which:

FIG.3A presents the result obtained after steps of: binding protein complex samples (10 $\mu$g each) to metal chelating agarose bead (lane 1); reducing the samples with 40 mM of 2-Mercaptoethanol at room temperature for 30 min (lane 2) ; oxidizing the protein complex with 5 mM of oxidized Glutathion form GSSG, for 2 hours at 37°C (lane 3); and separating 1/2 of the sample with non-reducing 8% SDS-Polyacrylamide gel, in which the closed arrows each indicate [B3(Fab)-ext-PE38]2, B3 (Fab) -ext-PE38 and B3 (Fd) -ext-PE38 from the top) The *open arrows* indicate TR proteins from complex.; and

FIG. 3B presents the result of separating the rest 1/2 of the sample with reducing 12% SDS-Polyacrylamide gel, in which the *two arrows* indicate B3(Fd)-ext-PE38 (*upper*) and H6-B3(L) (*bottom*). The *arrowheads* indicate TR proteins from complexes (TR1 protein (8kDa of molecular weight) does not appear in the drawing since the size of TR1 proteins is too small).

## DESCRIPTION OF THE PREFERRED EMBODIMENTS

Features and advantages of the present invention will be more clearly understood by the following detailed description of the present preferred embodiments by reference to the accompanying drawings. It is first noted that terms or words used herein should be construed as meanings or concepts corresponding with the technical sprit of the present invention, based on the principle that the inventor can appropriately define the concepts of the terms to best describe his own invention. Also, it should be understood that detailed descriptions of well-known functions and structures related to the present invention will be omitted so as not to unnecessarily obscure the important point of the present invention.

Hereinafter, the present invention is described in detail.

The present invention aims to produce large quantity of multimers formed by inter-monomer bond bridges and to provide a method for enhancing yield of bond bridged multimers by using repeat chains of affinity domains binding specifically to monomers as scaffolds, and producing complexes of repeat chain and monomers, thereby increasing local concentration of the monomers on the chains of the complexes, increasing frequency of collision between the monomers and facilitating formation of inter-monomeric bond bridges.

While bond bridged multimers are produced by linking multiple monomers with inter-monomeric bond bridges, the amount of produced bond bridged multimers depends on how the formation of bond bridges is efficient. For the substance to be used *in vivo*, the bridge bonds for forming multimers are preferred to be covalent bonds so that the multimers are not decomposed into monomers and the stability of multimers is maintained *in vivo*. In order for covalent bond bridges to be formed, the contact among the bridge bond forming chemical functional groups is required. However, the size of the chemical functional groups in protein is extremely smaller compared to the total size of protein macromolecule. Accordingly, when the macromolecules are moving freely in reaction solution, the provability of small chemical functional groups contacting each other is very low and mass formation of multimers is hardly possible unless there is a method for greatly increasing the contact of the functional groups.

In order to increase the formation of bond bridges between monomers, the present invention creates repeat chains of affinity domains binding specifically to monomers and binds the multiple monomers to the repeat chains making the distance between monomers within several nanometers, or tens of nanometers or hundreds of nanometers, which is the scale of molecular size. Therefore, the monomers cannot freely move in the solution. That is, the monomers are fixed in the limited space and contact each other by moving in a molecule sized space. Under this condition, local concentration of monomers is extremely high on each repeat chain to which multiple monomers are bound. Therefore, the frequency of contact between bound monomers is remarkably increased and the contact between chemical functional groups which can form bond bridges is also increased. Therefore, the formation of multimers is remarkably increased.

The target of the present invention may include not only disulphide covalent bond bridges linked to protein, but covalent bonds including amide bond, ester bond, glycosidic bond, or ether bond, linked to monomers of organic compounds, bio-molecules or proteins, or any other proteins. Also described herein is a method for improving formation efficiency of monomers, when the multimers are formed by inserting linker chains between monomers to form bridges between monomers producing a monomer-linker chain-monomer structure (Greg T. Hermanson, Bioconjugate Techniques. Academic Press, Inc., 1995; Wong S. S., Chemistry of Protein Conjugation and Cross-Linking. CRC Press, Inc.,

1991.)

More specifically, a method according to the present invention may preferably include the following steps of, but not limited thereto,:

1) preparing repeat chains of affinity domains binding specifically to monomers;
2) preparing repeat chain/multiple-monomer complexes of the repeat chains and monomers with mixing the repeat chains of step 1) and monomers; and
3) separating multimers in which bond bridges are formed between monomers within the repeat chain/multiple-monomer complexes of step 2), but not limited thereto.

According to the method, the monomers are proteins, and the affinity domains binding specifically to monomers of step 1) may preferably be originated from protein, but not limited thereto. That is, all kinds of molecules having specific binding affinity there between may be used as the affinity domains. For example, protein and small organic compound having specific binding affinity thereto may be used as the monomers and the affinity domains for a method according to the present invention.

According to the method explained above, the preparing repeat chains of affinity domains binding specifically to monomers (step 1) may include preparing repeat chains of affinity domains having different binding affinities together on the chain. Accordingly, hetero-dimer and hetero-multimer may be manufactured in which different monomers are specifically bound to different affinity domains.

According to the method explained above, in step 1), protein monomers and the protein domains having specific binding affinity to monomers thereto may preferably be originated from native form of protein, but not limited thereto. That is, if recombinant monomers have specific binding affinity to counterparts, the monomers may be used for producing multimers with bond bridges according to the method of the present invention. DNA fragments encoding gene information of the protein may preferably be manufactured by PCR cloning from chromosomal DNA or from mRNA of organisms expressing the proteins using forward and reverse pair of primer, but not limited thereto.

According to the method explained above, the bond bridges in step 3) may include not only the disulphide covalent bond bridges linked to protein, but also covalent bonds including amide bond, ester bond, glycosidic bond, or ether bond linked to monomers of organic compounds, bio-molecules or proteins.

According to the method explained above, the repeat chains of step 1) may be prepared by a method including steps of, but not limited thereto,:

1) preparing construct having repeated binding affinity proteins domains by repeatedly cloning DNA fragment encoding an affinity domain and a affinity domain connecting linker in an expression vector;
2) preparing transformant by transforming host cell with the construct of step 1) ; and
3) cultivating the transformant of step 2), and separating and purifying expressed repeat chain proteins with chromatography, but not limited thereto.

The construct may preferably have a structure in which the affinity domain is repeated over 3 times within repeat chains, but not limited thereto.

Regarding the construct, affinity domain may be linked by linker, and GGGGS may be used as G4S linker, but not limited thereto. The linker may be extended to, for example, GGGGSGGGGS or GGGGSGGGGSGGGGS, but also not limited thereto. The G4S linker has very flexible structure (R. Arai, et al., Protein Eng 14(2001) 529-532).

According to the present invention, plasmid pTR1 was prepared by cutting DNA fragments encoding affinity domain and linker with NdeI and EcoRI, and cloning the fragments on the vector part of pCW1 cut with the same enzymes.

The DNA fragments encoding affinity domain and G4S linker of pTR1 were cut out again with NdeI and BspEI to prepare DNA fragments of 226 bp encoding the DNA fragment of affinity domain and one G4S as a linker thereof, and plasmid pTR2 was prepared by cloning the prepared fragments onto the large vector fragment obtained by cutting the plasmid pTR1 with NdeI and AgeI. The above-mentioned cloning method was repeated 7 times and thus plasmid pTR7 was prepared, wherein the pTR7 has the construct in which affinity domains were repeated 7 times (See Table 1.). The proteins were over-expressed after transforming the E.coli BL21 (DE3), and separated and purified with chelating sepharose fast flow chromatography and size-exclusion chromatography.

According to the method, the monomers of step 2) have binding sequence (B) specifically binding to the affinity domain.

Regarding the formation of repeat chains-multiple monomers complex of step 2), it is possible to manufacture heterodimer and heteromultimer made of different monomers each specifically binding to each different affinity domains by using repeat chains of affinity domains having different binding specificity.

According to the method, inter-monomer counterpartless cysteine is preferably necessary to manufacture disulfide bond bridged multimers from a complex of multiple monomers of step 2), but not limited thereto. That is, various kinds of chemical cross-linker may be used for bond bridges between monomers as well as disulphide bond bridges between the counterpartless cysteines. If the monomer has any counterpartless cysteine for bond bridge, one counterpartless cysteine is preferably included therein, but not limited thereto.

According to the method, the monomers of step 2) may be used to make new kind of monomers by linking the monomers to functional proteins with extension sequence (Ext).

According to the method, functional proteins may include all of biological compound such as enzyme, toxin (functional) group protein or virus, pharmarceutical compound for drug activity, functional group such as liposomes, biosensor or prodrug.

The extension sequence (Ext) links monomers and functional proteins to fuse monomers and functional proteins and the extension sequence (Ext) may include counterpartless cysteine, but not limited thereto. The counterpartless cysteine is oxidized between the two monomers and forms disulphide bond bridges; therefore, dimers are formed. Also the extension sequence (Ext) includes flexible amino-acid sequence (Flx) between the last one of counterpartless cysteines of disulphide bond bridge dimer formation and hetero functional groups (F). The flexible sequence (Flx) is preferably comprised with sequence including GASQEND amino-acids (i.e., glycine, alanine, serine, glutamine, glutamic acid, asparagines and aspartic acid) which is not bulky amino-acid, but not limited thereto.

According to the method, the mixing of step 2) is preferably performed by mixing with the monomers and incubating 3 ~ 5 °C over night long and incubating at 35 ~ 40°C for 1 - 2 hrs. It is more preferable to perform by incubating 4°C over night long and incubating for 1 hr at 37 °C, but not limited thereto.

According to the method, the fixing of the complexes of step 3) is preferably performed by fixing the protein complex of the monomers bound to repeat chains onto metal chelating sepharose bead, but not limited thereto. When protein complex is fixed on the bead, it is preferably performed at 10 °C for 1 hr, but not limited thereto.

According to the method, regarding the formation of bond bridges, bond bridge-heteromultimer may be manufactured by forming bond bridges between different monomers in a complex of hetero-multiple monomers formed by repeat chains of affinity domains having different binding specificities.

According to the method, bond bridges of step 3) may be formed by using the various existing methods (Greg T. Hermanson, Bioconjugate Techniques. Academic Press, Inc., 1995; Wong S. S., Chemistry of Protein Conjugation and Cross-Linking. CRC Press, Inc., 1991.). If disulphide bond bridges are used, the functional group of counterpartless cysteine is reduced first. This reduction is preferably performed by adding the protein complex into reduction buffer solution including 2-merchaptoethanol, but not limited thereto. That is, regarding the reduction, the added amount of 2-merchaptoethanol is preferably 20 ~ 40 mM for full reduction of cysteine residues of monomers, but not limited thereto.

According to the method, if disulphide bond bridges are used to form bond bridges of step 3), multimers having disulphide bond bridges are produced by the oxidation of reduced cysteine residues of each monomer. The oxidation between the monomers is preferably performed by adding reduced protein complex into oxidation buffer solution including glutathione oxidized form (GSSG), but not limited thereto.

In the present invention, the reduction of protein complex is preferably performed by adding Tris-HCl (pH8.2) in which 2-merchaptoethanol is added, at room temperature, but not limited thereto. The reduced protein complex is preferably washed with the buffer solution including MOPS (pH6.5), but not limited thereto. The complex is washed 3 times more with Tris-HCl (pH8.2); oxidation buffer solution including GSSG and Tris-HCl (pH8.2) is added thereto for oxidation; and this is incubated at 37 °C for 2 hrs. According to the method, the temperature and time for incubation is preferably 37°C and for 2 hrs respectively, but not limited thereto.

According to the method, the analysis of bond bridge multimers of step 3) is preferably performed with SDS-PAGE (SDS-polyacrylamide gel electrophoresis), but not limited thereto. All methods which are commonly used for separation and purification of proteins may be used.

According to the method, multimers of step 3) are preferably the dimers in which monomers are linked by disulphide bonds, but not limited thereto. The monomers can be of fusion protein molecule in the form of 'binding domain (B)-extension sequence (Ext)-funtional protein (F)'. In case of the extension sequence includes counterpartless cysteine, disulphide bond bridges are formed between the counterpartless cysteine of two binding domains(B)-extension sequence (Ext)-functional protein(F) molecules; therefore, the dimers are formed in the form of [binding domain (B)-extension sequence (Ext)-functional proteins (F)]$_2$.

In order to complete the present invention, the inventors used antibody-toxin having Fab protein fragment of antibody as monomer's target binding domain (B) as in the form of 'binding domain (B)-extension sequence (Ext)-funtional protein (F)' monomer and Streptococcal protein G's Fab binding domain as affinity domain having specific binding affinity to Fab domain of antibody-toxin. All kinds of the antibody molecules (antibody derived molecule monomers) that contains Fab fragment of antibody as its part can be used as monomers as well as the ones in antibody-toxin form. In order to maximize the formation of dimers having disulphide bond bridges between monomers ([antibody derived molecule monomer]$_2$), recombinant repeat chains proteins are prepared wherein Fab binding domains of Streptococcal protein G are repeated over 2 times, and the prepared proteins are used as scaffold for preparing [antibody derived molecule monomer]$_2$. More specifically, the repeat chains and antibody derived molecule monomer proteins are mixed to manufacture the complex of recombinant repeat chains-antibody derived molecule monomer, in which many antibody monomers are bound to one recombinant chain protein with non-covalent bonds, and the cysteine residues of antibody monomers are reduced and oxidized to form the disulphide bond of cysteine residues located between Fab fragment and toxin of antibody derived molecule monomer, thus [antibody derived molecule monomers]$_2$ is prepared.

For comparing the formation level of [antibody derived molecule monomers]2 based on the present invention and the one of based on previous refolding method, the inventors of the present invention used SDS-PAGE and analyzed the yield of [antibody derived molecule monomers]2 through measuring the SDS-PAGE band density, and the result was that the yield of [antibody derived molecule monomers]2 based on the present invention is remarkably higher than the reported one performed based on the refolding method. That is, the yield of [antibody derived molecule monomers]$_2$ is in 36 to 52% range of the total antibody derived molecule monomers binding to recombinant repeat chain proteins wherein Fab binding domain of Streptococcal protein G is repeated over more than 2 times. Accordingly, it is confirmed that the yield range is about 144 to 208 times higher than the reported yield of existing refolding method.

As a result, it is confirmed that disulphide bond bridge dimers from bound antibody derived molecule monomers may be formed using repeat chains of Fab binding domain of protein G, and the use of the repeat chains increases the formation of [antibody derived molecule monomers]$_2$ drastically. By using repeat chains of Fab binding domains of protein G and its binding specificity to antibody molecule monomers, simple reduction and oxidation can produce large amount of [antibody derived molecule monomers]$_2$.

In addition, the present invention provides the gene construct encoding the expression of recombinant repeat chain protein of affinity domains binding specifically to monomers.

The present invention prepared the gene construct encoding recombinant repeat chain proteins of domain III (Fab binding domain) of Protein G.

The recombinant repeat chain proteins preferably include one linker, G4S (GGGGS), between two of Fab binding domains, but not limited thereto.

The recombinant repeat chain proteins preferably have the structure in which domain III (Fab binding domains) of protein G is repeated 3 to 7 times, but not limited thereto.

Also, the present invention provides expression vector including gene construct.

The expression vector is preferably connected to the desired gene of the specific nucleic acid sequencing having information for controlling gene transcription toward mRNA and translation to proteins to give good expression of desired protein and to increase the possibility of protein formation.

Furthermore, the present invention provides transformant produced by the expression vector in the host cells.

The inducing of the expression vector into host cells is performed by one of appropriate methods including transformation, transfection, conjugation, protoplast fusion, electroporation, calcium phosphate-precipitation or direct microinjection. The host cells may be prokaryotic or eukaryotic cells. Eukaryotic cells are preferable. Eukaryotic cells are the cells of mammals such as CHO cells. These cells are preferably the cell providing correct folding at correct position or transformation for protein molecule including glycosylation after generating the proteins, but not limited thereto.

The present invention confirmed that [antibody derived molecule monomers]$_2$ is able to be produced in large quantity from antibody derived molecule monomers with using recombinant repeat chain proteins as scaffold wherein Fab binding domain of Streptococcal protein G is repeated 3 to 7 times within the recombinant repeat chain proteins.

Accordingly, the repeat chains of affinity domains having specific binding affinity may be used to improve the formation of dimers and multimers between monomers. Also, gene construct encoding these repeat chains, expression vector including the gene construct and transformant transformed with the expression vector are very useful for mass formation of bond bridge multimers between antibody derived molecule monomers.

[References]

1. Pastan I. et al., Annu Rev Med. 58(2007) 221-237
2. L.H. Pai, et al., Proc Natl Acad Sci U S A 88(1991) 3358-3362
3. Pastan I. et al., Cancer Res. 51(1991) 3781-3787
4. J. Hwang, et al., Cell. 48(1987) 129-136
5. V.K. Chaudhary, et al., J Biol Chem 265(1990) 16306-16310
6. J. Buchner, et al., Biotechnology (N Y). 9(1991) 157-162
7. J. Buchner, et al., Biotechnology 10(1992) 682-685
8. M. Choe, et al., Cancer Res. 54(1994) 3460-3467
9. S.H. Choi, et al., Bull. Kor. Chem. Soc. 22(2001) 1361-1365
10. J.H. Park, et al., Mol Cells 12(2001) 398-402
11. M.H. Yoo, et al., J. Microbiol. Biotechnol. 16(2006) 1097-1103

12. D. Rothlisberger, et al., J Mol Biol 347(2005) 773-789

13. Korean Patent No. 10-0566091

14. Nicholas C. Price and Lewis Stevens. Fundamentals of Enzymology, 3rd Ed. Oxford University Press

15. R. Arai, et al., Protein Eng 14(2001) 529-532

16. Wong S. S., Chemistry of Protein Conjugation and Cross-Linking. CRC Press, Inc., 1991.

17. Greg T. Hermanson, Bioconjugate Techniques. Academic Press, Inc., 1995

18. T. Brody, et al., Anal Biochem 247(1997) 247-256

19. Nienhaus, G. Ulrich. Protein-ligand interactions : methods and applications. Humana Press , 2005

20. Farah, R. A., et al., Crit. Rev. Eukaryot. Gene Expr. 8, 321-356, 1998

21. Trail, P. A., et al., Science 261, 212-215, 1993

22. Hinman, L. M., et al., Cancer Res. 53, 3336-3342, 1993

23. Pastan, I. Biochim. Biophys. Acta 1333, C1-C6, 1997

24. Kreitman, P. J., et al., J. Clin. Oncol. 18, 1622-1636, 2000

25. Zalutsky, M. R. & Vaidyanathan, G. Curr. Pharm. Des. 6, 1433-1455, 2000

26. Goldenberg, D. M. in Clinical Uses of Antibodies (eds Baum, R. P., et al.)1-13(Kluwer academic, The Netherlands, 1991)

27. Lode, H. N. & Reisfeld, R. A. Immunol. Res. 21, 279-288, 2000

28. Penichet, M. L. & Morrison, S. L. J. Immmunol. Methods 248, 91-101, 2001

29. Lasic, D.D. & Papahadjopoulos, D. Science 267, 1275-1276, 1995

30. Park. J. W., et al., Proc. Natl Acad. Sci. USA 92, 1327-1331, 1995

31. Niculescu-Davaz, I., et al., Anticancer Drug Des. 14, 517-538,1999

32. SToldt, H. S., et al., Eur. J. Cancer 33, 186-192, 1997

33. Zachariou M., Affinity Chromatography: Methods and Protocols (Methods in Molecular Biology). Humana Press; 2nd edition.

Practical and presently preferred embodiments of the present invention are illustrative as shown in the following Examples.

### <Example 1> Preparation of repeat chains construct of Fab binding domain from Streptococcal protein G

The inventors obtained domain III of protein G from Korean Collection for Type Cultures (KCTC) and performed PCR cloning with P1[5'-GGGCA TATGC ATCAC CATCA CCATC ACACC GGTAC ACCAG CCGTG ACAA-3'(SEQ ID No: 1)] and P2[5'-CCCGA ATTCT TATCC GGACC CGCCT CCACC TTCAG TTACC GTAAA-3'(SEQ ID No:2)] primers from chromosomal DNA of Streptococcus. The PCR products (243 bp) were cut with NdeI and EcoRI, and were cloned into vector pCW1 which was cut with the same. The encoding sequence of the domain III was confirmed by dideoxy-DNA sequencing. G4S linker for each domain III was added as spacer and thus, pTR1 was obtained. The plasmid pTR1 was cut again with NdeI and BspEI and 225 bp fragment encoding domain III and one of G4S was ligated to the large fragment of identical pTR1 plasmid cut with NdeI and AgeI, thus pTR2 encoding the two time Tandem Repeat of domain III was obtained. The plasmid pTR2 was cut with NdeI and AgeI again, and the large fragment was ligated to 225 bp fragment to produce new plasmid 'pTR3'. As repeating the above-mentioned cloning method ten times, up to 10 repeat of domain III of protein G (pTR1 to pTR10) were prepared (See Table 1). Based on the existing method, pMC75H encoding H6-B3(L) was prepared (See KR 10-0566091).

[Table 1]

| Used plasmids and proteins in the present invention | | |
|---|---|---|
| Plasmid | Proteins | References |
| pCW1 | B3(Fd)-ext-PE38:Fd-SKPSIST-KASG$_4$C (G$_4$S)$_2$GGPE-PE38[a] | J.H. Park, et al., Mol Cells 12(2001) 398-402 |
| pMC75H | H6-B3(L): (His)$_6$ [b]-Light chain | The detailed description of the present invention |

(continued)

| Used plasmids and proteins in the present invention | | |
| --- | --- | --- |
| Plasmid | Proteins | References |
| pTR1~10 | TR1~10: $(His)_6$-$(D-G_4S)_n$, n=1~10[c] | The detailed description of the present invention |

[a]. SKPSIST: a modified sequence of wild-type hinge sequence(CKPCICT) ;
ext: SKPSIST-KASG4C(G4S)2GGPE: extended peptide chains having cystein residues (Cys residue);
G4S: amino acid sequence of GGGGS;
PE38: truncated Pseudomonas Exotoxin of 38 kD;
[b]. $(His)6$: 6 of Histidine tags; and
[c]. DIII: domain III of Streptococcal protein G.

Based on the existing method, the repeat chains were over-expressed (J.H. Park, et al., Mol Cells 12(2001) 398-402) .

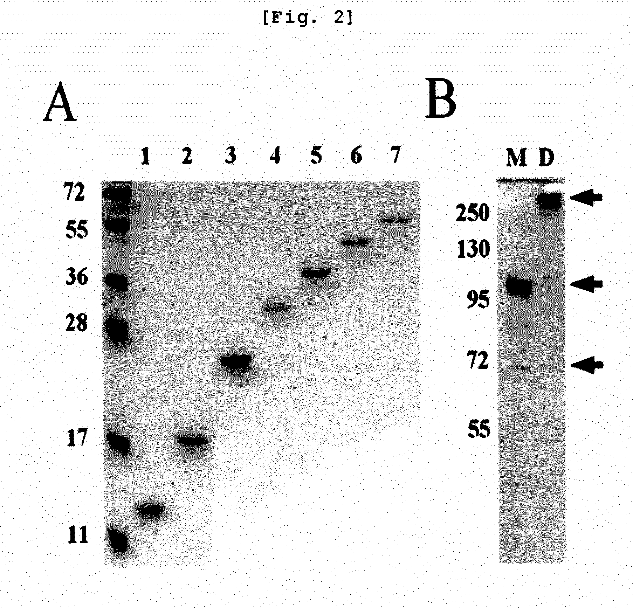

Pure lysate was separated with chelating sepharose fast flow chromatography (Amersham Bioscience, Sweden) and performed size-exclusion chromatography with Hiload Superdex-75 pg or Hiload Superdex-200 pg(26/60 (Amersham Bioscience, Sweden). Proteins of all the constructs were purified over 95% purity (See FIG.2A).

**<Example 2> Preparation of B3(Fab)-ext-PE38 and [B3(Fab)-ext-PE38] 2**

Based on the existing method, the inventors performed over-expression, preparation and refolding of inclusion body of B3(Fd)-ext-PE38 and H6-B3(L) (J.H. Park, et al., Mol Cells 12(2001) 398-402). The refolded proteins were purified with Q-sepharose FF, Hitrap protein G HP, and Hiload Superdex-200 pg(26/60)(Amersham Bioscience, Sweden) chromatography.

More specifically, B3(Fab)-ext-PE38 was prepared by inter-chain disulphide bond between B3(Fd)-ext-PE38 and H6-B3(L). The two cystein residues related to inter-chain disulphide bond locate one on CH1 domain of B3(Fd)-ext-PE38 and other on CL domain of H6-B3(L). Also, during the folding process, [B3(Fab)-ext-PE38]2 was produced by inter-monomeric disulphide bond between two of cystein residues located on the ext position of B3(Fab)-ext-PE38 monomer. After performing size-exclusion chromatography, the purities of B3(Fab)-ext-PE38 and [B3(Fab)-ext-PE38]2 were measured by using densitometric anlaysis of non-reducing SDS-polyacrylamide gel, and they showed over 95% purities (See FIG. 2B). The refolding yield of [B3(Fab)-ext-PE38]2 was about 0.06%.

**<Example 3> Binding of B3 (Fab) -ext-PE38 monomers with repeat chains construct from protein G domain III**

For the binding reaction between purified repeat chain construct of protein G domain III and B3(Fab)-ext-PE38, the inventors mixed B3 (Fab) -ext-PE38 (715 $\mu$g) and TR proteins (28 $\mu$g each), incubated at 4 °C over-night, and then incubated at 37 °C for 1 hr. The reacting mixture was separated by size-exclusion chromatography (Superdex-200TM HR). The elusion profile of the protein complex was compared to those of B3(Fab)-ext-PE38 only (Kav=0.33) or [B3(Fab)-ext-PE38]2 only (Kav=0.20) as controls. The Kav value of the eluted protein peak was calculated with [Formula 1].

【Formular 1】

$$Kav = \frac{(Ve-Vo)}{(Vt-Vo)}$$

wherein Ve is elution volume of the peak, Vo is void volume of the column, which is the elution volume of blue dextran 2000; and Vt is bed volume of superdex-200 column.

B3(Fab)-ext-PE38(Kav=0.22) bound to TR3 gave similar elution volume to that of [B3(Fab)-ext-PE38]2. Other complexes had 2 or more of B3(Fab)-ext-PE38 monomer molecules bound simultaneously to TR chains except TR2. TR3 to TR6 complexes were dimers of B3(Fab)-ext-PE38 monomer and TR7~10 complexes were trimers.

**<Example 4> Preparation of [B3(Fab)-ext-PE38]2 dimer from the complexes of repeat chains of protein G domain III and B3(Fab)-ext-PE38 monomers**

In order to produce [B3(Fab)-ext-PE38]2 dimer in the complexes of the purified TR 1~10 chain proteins and B3(Fab)-ext-PE38 monomers bound thereto, the inventors performed oxidation reactions to the bound monomer molecules on the chains with oxidized form of glutathione (GSSG), and the products were separated by non-reducing (FIG. 2A) and reducing (FIG. 2B) SDS-PAGE.

More specifically, The protein complex (10 μg) was mixed with metal-chelating Sepharose beads (20 μl) in a microtube for 1 h at 10 °C. The metal-chelating Sepharose beads were in a 50% suspension equilibrated with 100mM Tris-HCl buffer (pH 8.2). The immobilized protein complex was reduced by the addition of 40 mM 2-merchaptoethanol in 100 mM Tris-HCl buffer (pH 8.2) at room temperature. In a preparatory experiment for deciding the concentration of 2-Merchaptoethanol which was necessary to reduce cystein residues of B3(Fab)-ext-PE38, it was found that the full reduction of cystein residues was achived by adding 20 ~ 40 mM of 2-Merchaptoethanol. After the reduction, reduced protein complex was washed 1 time with washing buffer solution including 100 mM of MOPS (pH6.5) and washed 3 times more with 100 mM of TrisHCl (pH8.2). The washed protein complex was oxidized with oxidation buffer solution including 5 mM of GSSG and 100 mM of TrisHCl (pH8.2) and incubated at 37°C for 2 hrs. After the oxidation, 2X SDS sample buffer solution was added, the product was stained and analyzed and stained with SDS-PAGE and Coomassie. The yield of [B3(Fab)-ext-PE38]2 was analyzed through densitometry analysis that can measure the amount of protein on the SDS-PAGE samples. Using the data from electrophoresis of SDS-polyacrylamide gel (SDS-PAGE), the yield of [B3(Fab)-ext-PE38]2 was calculated with the band intensity of [B3(Fab)-ext-PE38]2 divided by total band intensity which was the sum of the intensity of [B3(Fab)-ext-PE38]2 and B3(Fab)-ext-PE38 (See Table 2.). B3(Fab)-ext-PE38 only and B3(Fab)-ext-PE38 bound to TR1 were used as controls.

[Table 2]

| The yield of [B3(Fab)-ext-PE38] 2 | |
|---|---|
| Antibody-toxin complexed with TR chains | The yield (%) |
| B3(Fab)-ext-PE38 | N/A |
| B3(Fab)-ext-PE38: TR1 | N/A |
| B3(Fab)-ext-PE38: TR2 | N/A |
| B3(Fab)-ext-PE38: TR3 | 47% ± 0.25 |
| B3(Fab)-ext-PE38: TR4 | 44% ± 0.19 |
| B3(Fab)-ext-PE38: TR5 | 48% ± 0.12 |
| B3(Fab)-ext-PE38: TR6 | 36% ± 0.02 |
| B3(Fab)-ext-PE38: TR7 | 52% ± 0.11 |

In the above experiment result, [B3(Fab)-ext-PE38]2 was not detected in 3 samples (i.e., B3(Fab)-ext-PE38 only, TR1 and TR2). Significant amount of inter-monomeric disulphide bond bridged dimers were produced by the interaction of monomers in the complexes with TR3~TR7 chains. In samples of TR3 ~ TR7 complexes, the yield of [B3(Fab)-ext-PE38]2 was 47%, 44%, 48%, 36% and 52%, respectively. [B3(Fab)-ext-PE38]2 was produced within 2 hrs after the addition of oxidizing agent and no significant increase was observed for extended incubation. It was confirmed that cystein residues in ext sequence of B3(Fab)-ext-PE38 molecule was have close contact to each other when they are bound to TR chains proteins. A thin band very close to [B3(Fab)-ext-PE38]2 was observed in non-reduction gel. It was confirmed that the thin band was another form of [B3(Fab)-ext-PE38]2 with incompletion of inter-chain disulphide bond between long heavy B3(Fd)-ext-PE38 chain and short light H6-B3(L) chain forming one B3(Fab)-ext-PE38 monomer without inter-chain disulphide bond, which loses one short light H6-B3(L) chain in non-reducing SDS-PAGE. It was already confirmed in the previous report about reduction decomposition studies on purified antibody (T. Brody, et al., Anal Biochem 247(1997) 247-256).

[Industrial Applicability]

The present invention relates to formation of multimers formed by bond bridges. With repeat chains of affinity domains having specific binding affinity, the formation of a repeat chain/multiple-monomer complex can be achieved, and thus a formed repeat chain/multiple-monomer complex can be used to produce multimers linked by bond bridges.

Remarkably high formation rate is provided by a method of the present invention and leads to formation of dimers linking with bond bridges in mass quantity; therefore, the present invention is industrially applicable.

Monomers can also be made with many kinds of protein substances including ligand, or a part fragment of the ligand having binding affinity such as TGF alpha, TGF beta, IL2, IL6, TNF or GMSCF, and various kinds of ligand receptors or a part fragment of ligand receptors having binding affinity such as TBP1, TBP2, IFN alpha or beta, or gonadotropin receptors. (Nienhaus, G. Ulrich. Protein-ligand interactions: methods and applications. Humana Press, 2005).

In addition, The followings may be used to form monomers: various kinds of enzymes catalyzing prodrugs transformation or detection, decomposition and formation of substance, proteins including functional group of cytotoxic toxin, [References: (Farah, R. A., et al., Crit. Rev. Eukaryot. Gene Expr. 8, 321-356, 1998) (Trail, P. A., et al., Science 261, 212-215, 1993) (Hinman, L. M., et al., Cancer Res. 53, 3336-3342, 1993) (Pastan, I. Biochim. Biophys. Acta 1333, C1-C6, 1997) (Kreitman, P. J., et al., J. Clin. Oncol. 18, 1622-1636, 2000) (Zalutsky, M. R. & Vaidyanathan, G. Curr. Pharm. Des. 6, 1433-1455, 2000) (Goldenberg, D. M. in Clinical Uses of Antibodies (eds Baum, R. P., et al.)1-13(Kluwer academic, The Netherlands, 1991)) (Lode, H. N. & Reisfeld, R. A. Immunol. Res. 21, 279-288, 2000) (Penichet, M. L. & Morrison, S. L. J. Immmunol. Methods 248, 91-101, 2001)(Lasic, D.D. & Papahadjopoulos, D. Science 267, 1275-1276, 1995) (Park. J. W., et al., Proc. Natl Acad. Sci. USA 92, 1327-1331, 1995) (Niculescu-Davaz, I., et al., Anticancer Drug Des. 14, 517-538,1999) (SToldt, H. S., et al., Eur. J. Cancer 33, 186-192, 1997)].

In nature, there are various kinds of substances having binding affinity relationship to each other. Ligands and acceptors, antibody and antigen, homodimer, heterodimer, or proteins forming multimers are the known substances. By using these substances having inter-binding-affinity as monomers and affinity domains, multimers linked with bond bridges may be prepared in large quantity from monomers based on a method of the present invention.

In the present invention, the repeat chains having specific binding affinity are characterized to form a repeat chain/multiple-monomer complex. Therefore, as fixing the repeat chains on resin, it is possible to perform affinity purification with high efficiency for the purification of useful protein molecules in monomeric or multimeric form in biotechnology and medical industry (Zachariou M., Affinity Chromatography: Methods and Protocols (Methods in Molecular Biology) Humana Press; 2nd edition).


## Claims

1. A method for producing multimers with covalent bond bridges, the method comprising steps of:

   1) preparing repeat chains of affinity domains having specific binding affinity to a protein monomer;
   2) preparing repeat chain/multiple protein monomer complexes by mixing the repeat chains and the protein monomers of step 1) and forming covalent bond bridges between the protein monomers; and
   3) separating multimers in which covalent bond bridges are formed between the protein monomers in the repeat chain/multiple protein monomer complexes of step 2), wherein the protein monomer contains chemical functional group to form covalent bond bridge.

2. The method as set forth in claim 1, wherein the protein monomers are antibodies, or fragments thereof, or recombinants thereof, or derivatives thereof, or fusions of biological or chemical functional group therewith, or wherein the monomers are ligands that bind to receptors or fragments thereof, or recombinants thereof, or derivatives thereof, or fusions of biological or chemical functional group therewith, or wherein the monomers are receptors or fragments thereof, or recombinants thereof, or derivatives thereof, or fusions of biological or chemical functional group therewith

3. The method as set forth in claim 2, wherein the protein monomers comprise fragments of antibodies, Fab fragments, or Fab containing fragments.

4. The method as set forth in claim 2, wherein the biological or chemical functional group comprises biological compounds comprising enzyme, toxin protein or virus, or pharmaceutical compound for drug activity, or functional group including liposome, bio-sensor or prodrug.

5. The method as set forth in claim 1, wherein the affinity domains are proteins.

6. The method as set forth in claim 1, wherein the affinity domains are proteins binding to the protein monomers.

7. The method as set forth in claim 1, wherein the affinity domains are proteins binding to antibody, to fragment of the antibody, or to Fab fragment.

8. The method as set forth in claim 1, wherein the affinity domain is protein G.

9. The method as set forth in claim 1, wherein the affinity domains are fragments of protein G.

10. The method as set forth in claim 9, wherein fragments of the protein G are antibody binding domains of protein G.

11. The method as set forth in claim 10, wherein the antibody binding domain of the protein G is domain III.

## Patentansprüche

1. Ein Verfahren zur Herstellung von Multimeren mit kovalenten Brückenbindungen, wobei das Verfahren die folgenden Schritte umfasst:

   1) Herstellen von sich wiederholenden Ketten der Affinitätsdomänen mit spezifischer Bindungsaffinität an ein Proteinmonomer;
   2) Herstellen von Komplexen aus sich wiederholenden Ketten und multiplen Proteinmonomeren durch Mischen der sich wiederholenden Ketten und der Proteinmonomeren aus Schritt 1) und Ausbilden von kovalenten Brückenbindungen zwischen den Proteinmonomeren; und
   3) Abtrennen der Multimere, bei denen sich kovalente Brückenbindungen zwischen den Proteinmonomeren in den Komplexen aus sich wiederholenden Ketten und multiplen Proteinmonomeren aus Schritt 2) gebildet haben, wobei die Proteinmonomere chemisch funktionelle Gruppen zur Ausbildung von kovalenten Brückenbindungen aufweisen.

**2.** Das Verfahren nach Anspruch 1, wobei die Proteinmonomere Antikörper, oder Fragmente davon, oder Rekombinante davon, oder Derivate davon, oder Fusionen mit biologischen oder chemischen funktionellen Gruppen sind, oder wobei die Monomere Liganden sind, die an Rezeptoren oder Fragmente, oder Rekombinante davon, oder Derivate davon, oder Fusionen mit biologischen oder chemischen funktionellen Gruppen binden, oder wobei die Monomere Rezeptoren oder Fragmente, oder Rekombinante davon, oder Derivate davon, oder Fusionen mit biologischen oder chemischen funktionellen Gruppen sind.

**3.** Das Verfahren nach Anspruch 2, wobei die Proteinmonomere Antikörperfragmente, Fab-Fragmente, oder Fab-enthaltende Fragmente umfassen.

**4.** Das Verfahren nach Anspruch 2, wobei die biologischen oder chemischen funktionellen Gruppen biologische Verbindungen umfassen, die ein Enzym, Toxin-Protein oder Virus, oder pharmazeutische Verbindungen mit Arzneimittelaktivität, oder funktionelle Gruppen einschließlich einem Liposom, Biosensor oder Arzneimittelvorläufer umfassen.

**5.** Das Verfahren nach Anspruch 1, wobei die Affinitätsdomänen Proteine sind.

**6.** Das Verfahren nach Anspruch 1, wobei die Affinitätsdomänen Proteine sind, die an die Proteinmonomere binden.

**7.** Das Verfahren nach Anspruch 1, wobei die Affinitätsdomänen Proteine sind, die an Antikörper, Antikörperfragmente oder Fab-Fragmente binden.

**8.** Das Verfahren nach Anspruch 1, wobei die Affinitätsdomäne Protein G ist.

**9.** Das Verfahren nach Anspruch 1, wobei die Affinitätsdomänen Fragmente von Protein G sind.

**10.** Das Verfahren nach Anspruch 9, wobei die Fragmente von Protein G Antikörperbindedomänen von Protein G sind.

**11.** Das Verfahren nach Anspruch 10, wobei die Antikörperbindedomäne von Protein G die Domäne III ist.

## Revendications

**1.** Procédé de production de multimères dotés de ponts à liaison covalente, le procédé comprenant des étapes consistant à :

1) préparer des chaînes récurrentes de domaines d'affinité ayant une affinité de liaison spécifique pour un monomère protéique ;
2) préparer des complexes chaîne récurrente - monomère protéique multiple en mélangeant les chaînes récurrentes et les monomères protéiques de l'étape 1) et en formant des ponts à liaison covalente entre les monomères protéiques ; et
3) séparer les multimères dans lesquels des ponts à liaison covalente sont formés entre les monomères protéiques dans les complexes chaîne récurrente-monomère protéique multiple de l'étape 2), le monomère protéique contenant un groupe fonctionnel chimique destiné à former le pont à liaison covalente.

**2.** Procédé ainsi que présenté dans la revendication 1, dans lequel les monomères protéiques sont des anticorps, ou des fragments de ceux-ci, ou des produits recombinés de ceux-ci, ou des dérivés de ceux-ci, ou des produits de fusion d'un groupe fonctionnel biologique ou chimique avec ceux-ci, ou dans lequel les monomères sont des ligands qui se lient à des récepteurs ou des fragments de ceux-ci, ou des produits recombinés de ceux-ci, ou des dérivés de ceux-ci, ou des produits de fusions d'un groupe fonctionnel biologique ou chimique avec ceux-ci, ou dans lequel les monomères sont des récepteurs ou des fragments de ceux-ci, ou des produits recombinés de ceux-ci, ou des dérivés de ceux-ci, ou des produits de fusion d'un groupe fonctionnel biologique ou chimique avec ceux-ci.

**3.** Procédé ainsi que présenté dans la revendication 2, dans lequel les monomères protéiques comprennent des fragments d'anticorps, des fragments Fab ou des fragments contenant Fab.

**4.** Procédé ainsi que présenté dans la revendication 2, dans lequel le groupe fonctionnel biologique ou chimique comprend des composés biologiques comprenant une enzyme, une protéine de toxine ou un virus, ou un composé pharmaceutique destiné à une activité médicamenteuse, ou un groupe fonctionnel incluant un liposome, un biocapteur ou un promédicament.

5. Procédé ainsi que présenté dans la revendication 1, dans lequel les domaines d'affinité sont des protéines.

6. Procédé ainsi que présenté dans la revendication 1, dans lequel les domaines d'affinité sont des protéines se liant aux monomères protéiques.

7. Procédé ainsi que présenté dans la revendication 1, dans lequel les domaines d'affinité sont des protéines se liant à un anticorps, à un fragment de l'anticorps, ou à un fragment Fab.

8. Procédé ainsi que présenté dans la revendication 1, dans lequel le domaine d'affinité est une protéine G.

9. Procédé ainsi que présenté dans la revendication 1, dans lequel les domaines d'affinité sont des fragments de protéine G.

10. Procédé ainsi que présenté dans la revendication 9, dans lequel les fragments de protéine G sont des domaines de la protéine G se liant à un anticorps.

11. Procédé ainsi que présenté dans la revendication 10, dans lequel le domaine de la protéine G se liant à un anticorps est le domaine III.

# [Fig.1]

recombinant repeat chain proteins + monomer proteins → mix → complexes of repeat chains and multiple monomers → bond bridge formation reaction → bond bridged multimers on repeat chains → elution and isolation

recovery and recycle of monomers ← monomers

recovery and recycle of repeat chains ← repeat chains

bond bridged multimers

• functional group for bond bridge (biological or chemical functional group)

[Fig. 2]

[Fig. 3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100566091

### Non-patent literature cited in the description

- **PASTAN I. et al.** *Annu Rev Med.,* 2007, vol. 58, 221-237
- **DEYEV et al.** *Nature Biotechnology,* 2003, vol. 21 (12), 1486-1492
- **L.H. PAI et al.** *Proc Natl Acad Sci U S A,* 1991, vol. 88, 3358-3362
- **I. PASTAN et al.** *Cancer Res.,* 1991, vol. 51, 3781-3787
- **J. HWANG et al.** *Cell,* 1987, vol. 48, 129-136
- **V.K. CHAUDHARY et al.** *J Biol Chem,* 1990, vol. 265, 16306-16310
- **J. BUCHNER et al.** *Biotechnology,* 1991, vol. 9, 157-162
- **J. BUCHNER et al.** *Biotechnology,* 1992, vol. 10, 682-685
- **M. CHOE et al.** *Cancer Res.,* 1994, vol. 54, 3460-3467
- **S.H. CHOI et al.** *Bull. Kor. Chem. Soc.,* 2001, vol. 22, 1361-1365
- **J.H. PARK et al.** *Mol Cells,* 2001, vol. 12, 398-402
- **M.H. YOO et al.** *J. Microbiol. Biotechnol.,* 2006, vol. 16, 1097-1103
- **D. ROTHLISBERGER et al.** *J Mol Biol,* 2005, vol. 347, 773-789
- **J.P. et al.** *Nature,* 1992, vol. 359, 752-754
- **NICHOLAS C. PRICE ; LEWIS STEVENS.** Fundamentals of Enzymology. Oxford University Press
- **GREG T. HERMANSON.** Bioconjugate Techniques. Academic Press, Inc, 1995
- **WONG S. S.** Chemistry of Protein Conjugation and Cross-Linking. CRC Press, Inc, 1991
- **R. ARAI et al.** *Protein Eng,* 2001, vol. 14, 529-532
- **PASTAN I. et al.** *Cancer Res.,* 1991, vol. 51, 3781-3787
- **T. BRODY et al.** *Anal Biochem,* 1997, vol. 247, 247-256
- **NIENHAUS, G. ULRICH.** Protein-ligand interactions : methods and applications. Humana Press, 2005
- **FARAH, R. A. et al.** *Crit. Rev. Eukaryot. Gene Expr.,* 1998, vol. 8, 321-356
- **TRAIL, P. A. et al.** *Science,* 1993, vol. 261, 212-215
- **HINMAN, L. M. et al.** *Cancer Res.,* 1993, vol. 53, 3336-3342
- **PASTAN, I.** *Biochim. Biophys. Acta,* 1997, vol. 1333, C1-C6
- **KREITMAN, P. J. et al.** *J. Clin. Oncol.,* 2000, vol. 18, 1622-1636
- **ZALUTSKY, M. R. ; VAIDYANATHAN, G.** *Curr. Pharm. Des.,* 2000, vol. 6, 1433-1455
- **GOLDENBERG, D. M. et al.** Clinical Uses of Antibodies. Kluwer academic, 1991, 1-13
- **LODE, H. N. ; REISFELD, R. A.** *Immunol. Res.,* 2000, vol. 21, 279-288
- **PENICHET, M. L. ; MORRISON, S. L.** *J. Immmunol. Methods,* 2001, vol. 248, 91-101
- **LASIC, D.D. ; PAPAHADJOPOULOS, D.** *Science,* 1995, vol. 267, 1275-1276
- **PARK. J. W. et al.** *Proc. Natl Acad. Sci. USA,* 1995, vol. 92, 1327-1331
- **NICULESCU-DAVAZ, I. et al.** *Anticancer Drug Des,* 1999, vol. 14, 517-538
- **STOLDT, H. S. et al.** *Eur. J. Cancer,* 1997, vol. 33, 186-192
- **ZACHARIOU M.** Affinity Chromatography: Methods and Protocols (Methods in Molecular Biology). Humana Press
- **NIENHAUS, G. ULRICH.** Protein-ligand interactions: methods and applications. Humana Press, 2005
- **NICULESCU-DAVAZ, I. et al.** *Anticancer Drug Des.,* 1999, vol. 14, 517-538